# EUROPEAN PATENT APPLICATION

(11) **EP 2 510 957 A2**
(43) Date of publication of application: **17.10.2012**
(21) Application number: 10836194.0
(22) Date of filing: 07.12.2010
(51) Int. Cl.: A61L 27/04, A61L 27/50, A61L 27/06

(54) **IMPLANT**

(30) Priority: 07.12.2009 KR 20090120346
(71) Applicant: U & I Corporation, Uijeongbu-si Gyeonggi-do 480-050 (KR)
(72) Inventor: KOO, Ja-Kyo, Seoul 139-784 (KR); SEOK, Hyun-Kwang, Seoul 135-283 (KR); YANG, Seok-Jo, Daegu 704-700 (KR); KIM, Yu-Chan, Seoul 142-100 (KR); CHO, Sung-Youn, Uijeongbu-si Gyeonggi-do 480-901 (KR); KIM, Jong-Tack, Jeonju-si Jeollabuk-do 561-803 (KR)
(74) Representative: Collin, Jérôme
(86) International application number: PCT/KR2010/008717
(87) International publication number: WO 2011/071299

(57) **Abstract**

The present invention provides an implant, which is used to fix an artificial joint in bone, including a biodegradable magnesium alloy.

## Description

### CROSS REFERENCE TO RELATED APPLICATION

This application claims the benefit of Korean Patent Application No. KR 10-2009-0120346, filed December 7, 2009, which is hereby incorporated by reference in its entirety into this application.

### BACKGROUND OF THE INVENTION

### 1. Technical Field

The present invention relates to an implant.

### 2. Description of the Related Art

A function of artificial joints (for example, hip joint implants, knee joint implants, etc.) is to grow bone tissues when they are transplanted into a bone. In this case, bone tissues grow and thus the artificial joint is fixed in the bone. In conventional artificial joints, an artificial joint is coated with bone cement or hydroxyapatite (HA) in order to improve the interfacial force between the bone tissue and the artificial joint. Here, in the case of bone cement, the bone cement is kneaded and then adhered to an artificial joint at the time of the operation. The bone cement can be broken after an operation, and the eluted substances and particles of the bone cement can have a harmful effect on the performance of an artificial joint and the human body. In particular, it has been reported that the particles of the bone cement block pulmonary blood vessels to cause death. Further, in the case of hydroxyapatite (HA), the interfacial force between HA and bone is improved to increase the bonding force between bone and an implant. However, there is a problem in that HA separates from an artificial joint.

Meanwhile, a screw, which is made of only a biodegradable polymer, has been used as a fixing implant for improving the bone union of an artificial joint, but is problematic in that it has insufficient mechanical strength and is uniformly decomposed. For example, Japanese Unexamined Patent Publication Nos. 2002-253665 and 2000-070278 disclose a screw for fixing an artificial joint, the screw being made of a biodegradable polymer.

In addition to such an implant for fixing an artificial implant, Japanese Unexamined Patent Publication Nos. 1993-049657 discloses a biodegradable tool for preventing an artificial joint from being moved, the tool serving to improve the bone union of an artificial joint.

Therefore, in order to overcome or minimize the above-mentioned problems and to improve the bone union of a conventional biodegradable implant, it is required to develop various methods of increasing the bonding force between an implant and bone.

### SUMMARY OF THE INVENTION

Accordingly, the present invention intends to provide the following implants: i) an implant which has a high initial strength, and the rigidity (elastic coefficient) of which is similar to that of bone; ii) an implant which has an initial bending strength and pullout strength higher than those of bone and which maintains the initial bending strength and pullout strength until an artificial is fixed in bone; iii) an implant, the decomposition rate of which can be controlled such that 60% or more of its initial mechanical properties are retained until the implant has cured after an artificial joint has been fixed on bone using the implant, which is uniformly decomposed from the surface thereof to the inside thereof, and which does not cause a poisonous tissue reaction (for example, inflammation); iv) an implant which has no side effects due to itself and the decomposed products thereof; v) an implant wherein bone cells permeate into the vacant space of the implant when the implant decomposes and is absorbed in bone, so replacing the implant by bone; and vi) an implant which can be strongly fixed around bone.

In order to accomplish the above object, the present invention provides an implant including a biodegradable magnesium alloy, the implant being used to fix an artificial joint in bone.

### BRIEF DESCRIPTION OF THE DRAWINGS

FIG. 1 is a perspective view showing an implant and an artificial joint according to an embodiment of the present invention.

FIG. 2 is a photograph showing a screw type implant (arrow) of Example 1 mounted on an artificial joint.

FIG. 3 shows photographs of the test sample dyed with hematoxylin-eosin (H&E) 1 week, 8 weeks and 12 weeks after the implant of Example 2 was inserted into the thighbone of a rat.

FIG. 4 shows histograms analyzing the appearance of alkaline phosphatase (ALP) and osteocalcin (OC) indicating a bone forming index and a bone growth index using the RNA which was extracted from the bone sample 1 week, 2 weeks, 4 weeks, 8 weeks and 12 weeks after inserting the implant of Example 2 into the thighbone of a rat.

FIG. 5 shows photographs of the surface of the biodegradable magnesium alloy of Preparation Example 2.

### DESCRIPTION OF THE PREFERRED EMBODIMENTS

Hereinafter, preferred embodiments of the present invention will be described in detail.

I. Implant

The implant of the present invention is used to fix an artificial joint in bone, and includes a biodegradable magnesium alloy. The implant serves to fix an artificial joint in bone at the time of the administration of the artificial joint. With the passage of time, the implant is decomposed in bone to induce the formation of bone. The region in which the implant is decomposed is filled with bone, and thus the implant improves the adhesion between the artificial joint and the bone. In this case, the decomposition rate of the implant and the formation rate of the bone can be adjusted by controlling the amount of a biodegradable magnesium alloy.

The shape of the implant is not particularly limited as long as the implant can fix an artificial joint in bone, but it is preferred that the shape thereof be selected from a screw, a bolt, a pin, a nail and a washer. Here, the screw may be a cannula-type screw. The washer is used to disperse the load of a pin or the like.

The artificial joint is not particularly limited as long as it can replace joints of a human body, but it is preferred that the artificial joint be selected from the group consisting of an artificial hip joint, an artificial knee joint, an artificial shoulder joint, an artificial elbow joint, an artificial wrist joint, an artificial ankle joint, an artificial finger joint and an artificial toe joint.

The biodegradable magnesium alloy may be represented by the following Formula 1:

Mg-A (1)

wherein A is at least one selected from the group consisting of manganese (Mn), cobalt (Co), nickel (Ni), chromium (Cr), copper (Cu), cadmium (Cd), zirconium (Zr), silver (Ag), gold (Au), palladium (Pd), platinum (Pt), rhenium (Re), iron (Fe), zinc (Zn), molybdenum (Mo), niobium (Nb), tantalum (Ta), titanium (Ti), strontium (Sr), silicon (Si), phosphorus (P) and selenium (Se).

More preferably, the biodegradable magnesium alloy may be represented by the following Formula 2:

MgₐCa_{b}X_{c} (2)

wherein a, b and c are each independently a molar ratio of each component, a+b+c=1, 0.5≤a<1, 0≤b≤0.4, 0≤c≤0.4, and when at least one of b and c is more than 0 and c is 0, calcium (Ca) is included in an amount of 5 - 33 wt% based on a total amount of the biodegradable magnesium alloy; and X is at least one selected from the group consisting of zirconium (Zr), molybdenum (Mo), niobium (Nb), tantalum (Ta), titanium (Ti), strontium (Sr), chromium (Cr), manganese (Mn), zinc (Zn), silicon (Si), phosphorus (P), nickel (Ni) and iron (Fe).

Even when X includes two or more kinds, the molar ratio of X should be within the range of c. As the amounts of Ca and X increase, the strength and decomposition rate of a biodegradable magnesium alloy increase. Therefore, in the implant of the present invention, the amounts of Ca and X can be determined within the above range in consideration of the strength of the biodegradable magnesium alloy and the disappearance rate of the metal charged in the biodegradable magnesium alloy.

When X includes nickel (Ni), nickel (Ni) serves to reduce the toxicity of a biodegradable implant and to easily control the corrosion rate thereof. In this case, the amount of nickel (Ni) may be 100 ppm or less, and preferably 50 ppm or less. Further, when X includes iron (Fe), iron (Fe) greatly influences the increase of the corrosion rate of the biodegradable magnesium alloy. Therefore, the amount of iron (Fe) may be 1,000 ppm or less, and preferably 500 ppm or less. In this case, when the amount of iron (Fe) is more than 1,000 ppm, iron (Fe) exists as an independent factor because it cannot be solid-dispersed in magnesium (Mg), thus increasing the corrosion rate of magnesium (Mg).

The biodegradable magnesium alloy may include magnesium (Mg) and an impurity; the impurity may be any one selected from the group consisting of manganese (Mn), iron (Fe), nickel (Ni) and mixtures thereof, and the amount of the impurity may be 1 part by weight or less but exceeding 0 parts by weight based on 100 parts by weight of magnesium (Mg), and also the ratio of any one selected from the group consisting of iron (Fe), nickel (Ni) and mixtures thereof to manganese (Mn) may be 5 or less but exceeding 0 .

Preferably, the ratio of any one selected from the group consisting of manganese (Mn), iron (Fe), nickel (Ni) and mixtures thereof to manganese (Mn) may be 0.2 or less but exceeding 0. When the ratio thereof is within the above range, the decomposition rate of the implant in a living body becomes extremely low, thus improving the corrosion resistance of the implant. For this reason, the implant can stay in the living body for a long period of time.

When the impurity includes nickel (Ni) and manganese (Mn), the nickel (Ni) causes an allergic reaction to a human body and increases the corrosion rate of pure magnesium (Mg). Therefore, the amount of nickel (Ni) may be 100 ppm or less, and preferably 5 0 ppm or less.

The biodegradable magnesium alloy is represented by the following Formula 3, and may include 23 wt% or less but exceeding 0 wt% of Ca; 10 wt% or less but exceeding 0 wt% of Y; and a balance of Mg:

Mg-Ca-Y (3)

wherein Y is Mn or Zn.

When the biodegradable magnesium alloy represented by Formula 3 above is within the above range, there can be provided a biodegradable implant which has improved mechanical properties and corrosion resistance and which does not cause brittle fracture.

Further, the biodegradable magnesium alloy represented by Formula 3 above may include 23 wt% or less but exceeding 0 wt% of Ca, 0.1 ~ 5 wt% of Y, and a balance of Mg. More preferably, the biodegradable magnesium alloy may include 23 wt% or less but exceeding 0 wt% of Ca, 0.1 ~ 3 wt% of X, and a balance of Mg. The reason for this is that, due to the side effects of the impurity, a small amount of the impurity is advantageous provided that the corrosion rate stays the same.

Further, the biodegradable implant of the present invention may include a magnesium composite including the biodegradable magnesium alloy and at least one selected from the group consisting of metal, ceramic and polymer.

Further, the biodegradable magnesium alloy included in the implant of the present invention may be surface-coated. When the biodegradable magnesium alloy is surface-coated, corroded products are formed on the surface thereof, thus decreasing the decomposition rate thereof.

The biodegradable magnesium alloy may be surface-coated with a ceramic and/or a polymer.

First, the surface coating of the biodegradable magnesium alloy using ceramic is described. When the biodegradable magnesium alloy is immersed in a saline solution or biomimetic liquid, corroded products are formed on the surface of the biodegradable magnesium alloy, and the surface thereof can be coated with these corroded products. Here, the corroded product is ceramic, and the ceramic may be magnesium oxide or calcium phosphate. The surface of the biodegradable magnesium alloy is coated with the corroded product and then further coated with a polymer. The kinds of the polymer are as follows.

The polymer used to coat the surface of the biodegradable magnesium alloy is not particularly limited as long as it can be used in the related field. Preferably, the polymer may be poly(L-lactide), poly(glycolide), poly(DL-lactide), poly(dioxanone), poly(DL-lactide-co L-lactide), poly(DL-lactide-co-glycolide), poly(glycolide-co-trimethylene carbonate), poly(L-lactide-co-glycolide), poly(e-caprolactone) or a combination thereof

The biodegradable magnesium alloy included in the implant of the present invention may be surface-treated. Preferably, the surface treatment of the biodegradable magnesium alloy may be conducted by shot peening or sand blasting.

The biodegradable magnesium alloy may be ultrasonically treated. When the biodegradable magnesium alloy is ultrasonically treated, there is an advantage in that the corrosion rate thereof in the living body increases, and thus the implant disappears in a short period of time.

In the biodegradable implant according to another embodiment of the present invention, the biodegradable magnesium alloy may be formed into a porous structure, or may be charged in a porous structure.

The implant of the present invention may further include a porous structure in addition to the biodegradable magnesium alloy.

The pore size of the porous structure may be adjusted by any method commonly used in the related field. Preferably, the pore size thereof may be 200 ~ 500 *µ*m. When the pore size thereof is within the above range, blood vessels for supplying nutrients, minerals and ions can easily pass through pores.

It is preferred that the porosity of the porous structure be 5 ~ 95%. Here, the porosity is defined as "a volume ratio of pores in the porous structure". When an object to be applied requires high strength, the strength of the porous structure can be increased by decreasing the porosity thereof. Specifically, for example, when the porous structure is made of a metal having high strength, such as tantalum, or when the porous structure serves to fill the cavities of lost bone, the porosity thereof may be increased as well.

The porous structure may be made of at least one of a metal, a ceramic and a polymer. When the porous structure is made of a metal, the metal may be at least one selected from the group consisting of titanium, titanium alloys, cobalt-chromium alloys, and stainless steel. When the porous structure is made of a ceramic, the ceramic may be at least one selected from the group consisting of calcium phosphate, alumina, zirconia, and magnesia. When the porous structure is made of a polymer, the polymer may be at least one selected from the group consisting of polyethylene (PE), polylactic acid (PLA), polyglycolic acid (PGA), a copolymer (PLGA) of PLA and PGA, poly(L-lactide), poly(glycolide), poly(DL-lactide), poly(dioxanone), poly(DL-lactide-co L-lactide), poly(DL-lactide-co-glycolide), poly(glycolide-co-trimethylene carbonate), poly(L-lactide-co-glycolide), poly(e-caprolactone), and combinations thereof Here, when the porous structure is made of the above polymer, biodegradable acid is produced, and thus the pH becomes low. In this case, when the porous structure is a polymer composite material whose pores are filled with biodegradable magnesium, there is an effect of increasing pH while decomposing magnesium. Therefore, if controlling the decomposition rates of polymer and magnesium, the additional effect of controlling the pH in the living body can be anticipated.

Further, the implant of the present invention may further include a material which helps bone cells to attach to the implant or which can induce or accelerate the formation of blood vessels or bone. Examples of the material may include at least one selected from the group consisting of BMP(bone morphogenic protein)-2, BMP-4, BMP-6, BMP-7, IGF(insulin like growth fadtor)-1, IGF-2, FGF(fibroblast growth factor), PDGF(platelet derived growth factor), TGF(transforming growth factor)-β, VEGF (vascular endothelial growth factor), fibronectin, and vitronectin.

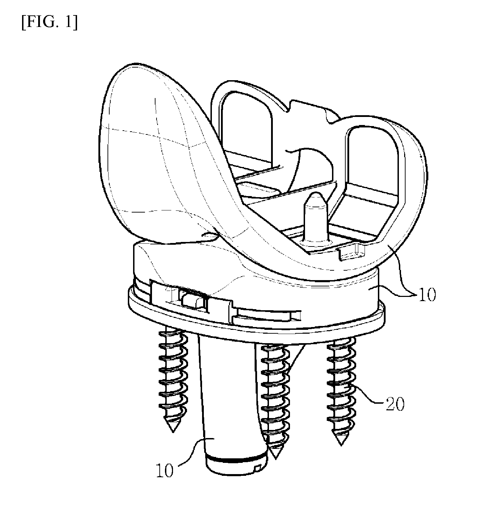

Meanwhile, FIG. 1 is a perspective view showing an implant and an artificial joint according to an embodiment of the present invention.

Referring to FIG. 1, in order to fix an artificial joint 10 in bone (not shown), the implant 20 according to an embodiment of the present invention is bonded with the artificial joint 10. The implant 20 is formed in the shape of a screw, but is not limited thereto.

II. Method of manufacturing an implant

The method of manufacturing an implant according to an embodiment of the present invention includes the steps of: a) providing a biodegradable magnesium alloy; and b) forming the biodegradable magnesium alloy.

In step a), the biodegradable magnesium alloy is melted. More concretely, in step a), the biodegradable magnesium alloy may be melted under an inert gas atmosphere (argon (Ar) which does not react with magnesium) or under a vacuum atmosphere. Further, in step a), the biodegradable magnesium alloy may be melted using various methods such as a resistance heating method for generating heat by applying electric current to a resistor, an induction heating method of generating heat by applying electric current to an induction coil, a heating method of generating heat using a laser or collected light, and the like. Here, among these melting methods, the resistance heating method is most efficient. In the procedure of melting the biodegradable magnesium alloy, it is preferred that the molten biodegradable magnesium alloy be stirred in order to uniformly mix impurities in the biodegradable magnesium alloy.

When the implant according to another embodiment of the present invention includes a porous structure whose pores are filled with the biodegradable magnesium alloy, the step a) may include the steps of: a-1) providing a porous structure; and a-2) filling the pores of the porous structure with the biodegradable magnesium alloy.

In step a-1), the porous structure may be made of any one selected from the group consisting of a metal, a ceramic and a polymer.

When the porous structure is made of only a metal, the step a-1) is as follows.

First, a metal is formed into metal powder or metal wire. The metal powder or metal wire is prepared into a green preform. The method of preparing the preform may be performed using sintering or modified sintering.

The method of preparing a preform using sintering is described as follows.

First, the metal powder or metal wire is put into a container or is pressed at a pressure of 100 MPa or less to allow the metal powder or metal wire to have low strength. The metal powders or metal wires having low strength are left at a temperature of 2/10 ~ 9/10 of the melting point of the metal to be bonded with each other, thereby preparing a preform having mechanical strength.

Further, the method of preparing a preform using modified sintering is described as follows.

When the metal powder or metal wire is put into a conductive container made of graphite and then high current is applied to the conductive container, heat is instantaneously generated from the contract region of the metal powder or metal wire to form a sintered body, thereby preparing a preform.

When the porous structure is made of a metal and a polymer, the step a-1) is as follows.

First, a metal is formed into metal powder or metal wire. Subsequently, when the metal powder or metal wire is mixed with a polymer and is then heated, the polymer is decomposed at low temperature, and the metal powder or metal wire is sintered at high temperature, thereby preparing a preform having suitable mechanical strength. In this case, the porosity and strength of the preform are determined depending on sintering temperature, pressure, a mixing ratio of polymer and metal, and the like, and, if necessary, these conditions may be properly changed. The sintering temperature is changed depending on the kind of the raw material of a porous structure, and, generally, may be 1/2 ~ 9/10 of the melting point of the porous structure. Although the metal powder or metal wire is sintered even when pressure is not applied, it is rapidly sintered as the pressure becomes high. However, since additional costs, such as an equipment cost, a mold cost and the like, are required as the pressure becomes high, it is preferred that a suitable pressure be selected.

Further, in addition to the above method, when the porous structure is made of a metal and a polymer, the step a-1) is as follows.

First, the surface of a polymer is plated with a precious metal such as gold (Au), platinum (Pt), palladium (Pd) or the like. Subsequently, the polymer is removed, thus forming a biocompatible porous metal structure.

When the porous structure is made of a water-soluble salt and a metal, the step a-1) is as follows.

First, a water-soluble salt is mixed with metal powder, and then the mixture is formed at high temperature to prepare a preform. Here, the water-soluble salt may be at least one selected from the group consisting of NaNO₂, KNO₂, NaNO₃, NaCl, CuCl, KNO₃, KCl, LiCl, KNO₃, PbCl₂, MgCl₂, CaCl₂ and BaCl₃. Subsequently, the preform is pressed at a temperature of 2/10 ~ 9/10 of the melting point of the metal powder. In the procedure of pressing the preform, metal powders are bonded with each other by atom transfer to form a composite material containing the water-soluble salt. When the composite material is immersed in water, only the water-soluble salt oozes out of the composite material, thus preparing a porous metal structure.

When the porous structure is made of a polymer and an electrolyte containing metal ion, the step a-1) is as follows.

First, the surface of a porous polymer is plated with a metal using an electrolyte containing the metal ion. In this case, the metal is not particularly limited, but may be at least one selected from the group consisting of Ti, Co, Cr and Zr. Subsequently, the polymer is removed by heating, thus preparing a porous metal structure.

When the porous structure is made of a ceramic, the step a-2) is as follows.

First, ceramic powder is mixed with a binder (polymer). The mixture is applied onto the surface of foam made of a removable material such as polyurethane and is then dried to form a porous structure. Thereafter, when the porous structure is heated, the polymer is burned around the combustion temperature thereof to be removed. Subsequently, when the porous structure is further heated, the remaining ceramic powder is sintered, thus preparing a porous structure having mechanical strength.

Here, the ceramic powder may be at least one selected from the group consisting of hydroxyapatite (HA) powder, zirconia powder and alumina powder.

In step a-1), a porous structure whose inner and outer porosities are different from each other may also be prepared by modifying or combining the above-mentioned methods or by using a method of preparing a porous structure using different kinds of materials. In the latter method, a porous structure having different porosity with respect to position, the inside of which is not porous or has low porosity and the outside of which is highly porous, can be prepared.

In step a-2), a method of immersing the porous structure in the molten biodegradable magnesium alloy, a method of filling the pores of the porous structure with the molten biodegradable magnesium alloy while fixing the porous structure, or a method of easily filling the pores of the porous structure with the molten biodegradable magnesium alloy by externally applying a pressure of 1 atm or more may be used. In this case, in order not to solidify the molten biodegradable magnesium alloy while it is being charged into the pores of the porous structure, the porous structure may be heated or contaminants may be removed from the porous structure such that the molten biodegradable magnesium alloy is easily charged in the pores of the porous structure.

More concretely, in step a-2), first, magnesium is gasified at a high temperature of 700°C or more, and then the magnesium gas is deposited on the surface of pores of the porous structure while passing through the pores thereof, thus filling the pores of the porous structure with magnesium.

Further, more concretely, in step a-2), a magnesium-containing salt is dissolved in liquid, and then the porous structure passes through the solution, and thus magnesium is adsorbed on the surface of the pores of the porous structure.

In addition to the above methods of filling the magnesium alloy in the pores of the porous structure, in another modified example thereof, the pores of the porous structure may be partially, not completely, filled with the magnesium alloy. That is, when the porous structure is filled with the molten magnesium, and then high-pressure gas is blown into the porous structure or the porous structure is rotated or shaken before the molten magnesium has completely been solidified, the non-solidified magnesium is removed from the porous structure, and a part of magnesium remains in the pores of the porous structure, thereby preparing a composite material whose pores are partially impregnated with magnesium. In this case, the filling rate of magnesium may be different depending on the position of the pores of the porous structure.

Meanwhile, in the case of a polymer whose melting point is lower than that of magnesium, when a porous polymer structure is formed and then filled with molten magnesium, the shape of the porous polymer structure cannot be maintained. Therefore, it is preferred that an implant including a biodegradable magnesium alloy be manufactured by mixing a biodegradable magnesium alloy powder with a polymer at a mixing ratio of 5:95 ~ 95:5, heating the mixture to a temperature of 150 ~ 500°C and then pressing the mixture at a pressure of 1 ~ 100 atm. The above conditions are used only to manufacture an implant including a biodegradable magnesium alloy, and the implant can be manufactured under conditions deviating from these conditions. Therefore, the patent right of the present invention cannot be infringed by changing the conditions.

The above-mentioned methods, such as the method of preparing a porous structure using a metal, ceramic or polymer, the method of filling the pores of a porous structure with a magnesium alloy and the method of manufacturing an implant including a biodegradable magnesium alloy, are set forth to illustrate the present invention, and the scope of the present invention is not limited thereto.

In the method of manufacturing an implant according to an embodiment of the present invention, in step b), the molten biodegradable magnesium alloy may be formed by at least one of a cooling process, an extrusion process and a metal working process.

The shape of the implant obtained by forming the molten biodegradable magnesium alloy is not particularly limited as long as it can be used to fix an artificial joint in bone, but may be selected from the group consisting of a screw, a bolt, a pin and a nail.

In the forming of the molten biodegradable magnesium alloy, a cooling process can be used to improve the mechanical strength of a magnesium alloy. More concretely, when magnesium is melted in step a), the cooling process may be performed by immersing the crucible including the molten magnesium into water. Further, the cooling process may also be performed by spraying the molten magnesium with an inert gas (argon or the like). In the spraying, since the molten magnesium is rapidly cooled, very fine tissues can be obtained. However, in the spraying, there is a problem in that many pores can be formed in the porous structure when magnesium is cast in a small size.

The extrusion process is used to make the tissue of the magnesium alloy uniform and to improve the mechanical performance of the magnesium alloy. The extrusion process may be performed at a temperature of 300 ~ 450°C. Further, the extrusion of the magnesium alloy may be performed such that the reduction ratio (extrusion ratio) of the section areas of the magnesium alloy before and after the extrusion is 10:1 ~ 30:1. As the extrusion ratio increases, there is an advantage in that the fine tissues of the extrude product become uniform and in that the defects occurring during casting are easily eliminated. In this case, the capacity of an extruding machine must be increased.

The metal working process is not particularly limited as long as it is commonly known to those skilled in the art. Examples of the metal working process may include a process of pouring a molten magnesium alloy into a frame having a shape similar to that of a final product and directly casting the molten magnesium alloy, a process of forming a molten magnesium alloy into a rod-shaped or plate-shaped intermediate material and then milling the intermediate material, a method of pressure-forging a magnesium alloy and then forming the magnesium alloy into a final product, and the like.

Meanwhile, the present inventors verified that, when the biodegradable implant of the present invention is directly inserted into bone, the implant is biologically decomposed, and simultaneously bone tissues grow in the decomposed implant, so that the strength of bone can be enhanced, and the lost bone can be recovered.

Hereinafter, the present invention will be described in more detail with reference to the following Examples. These Examples are set forth to illustrate the present invention, and the scope of the present invention is not limited thereto.

<Preparation Examples>

The components having composition ratios given in Table 1 below were charged into a crucible made of stainless steel (SUS 410) and having an inner diameter of 50 mm. Subsequently, The crucible was heated to a temperature of 700°C ~ 750°C using a resistance heating furnace while argon gas flowing around the crucible such that iron, nickel, manganese, aluminum and magnesium charged in the crucible did not come into contact with air, so as to melt iron, nickel, aluminum, manganese and magnesium. The molten iron, nickel, aluminum, manganese and magnesium were stirred by shaking the crucible such that they were easily mixed with each other. The completely-molten magnesium alloy was cooled to prepare a solid magnesium alloy. In this case, in order to improve the mechanical strength of the solid magnesium alloy, the molten magnesium alloy was rapidly cooled by immersing the crucible into water (20°C).

**[Table 1]**

| | Fe (parts by weight) | Ni (parts by weight) | Al (parts by weight) | Mn (parts by weight) | Mg (parts by weight) | (Fe+Ni)/Mn | Ca (parts by weight) |
|---|---|---|---|---|---|---|---|
| Prep. Exp. 1 | 0.0014 | 0.0002 | 0.0021 | 0.0015 | 100 | 2.26 | - |
| Prep. Exp. 2 | - | - | - | - | 95 | - | 5 |

<Example 1>

The biodegradable magnesium alloy of Preparation Example 1 was pulverized to obtain powder having an average particle size of 500*µ*mu (hereinafter, referred to as "biodegradable magnesium alloy powder").

The biodegradable magnesium alloy powder was mixed with a biodegradable polymer (LPLG (poly(L-lactide-co-glycolide))) at a volume ratio of 85:15. Thereafter, the mixture was pressed at a temperature of 200°C and a pressure of 5 atms to manufacture a screw-shaped implant.

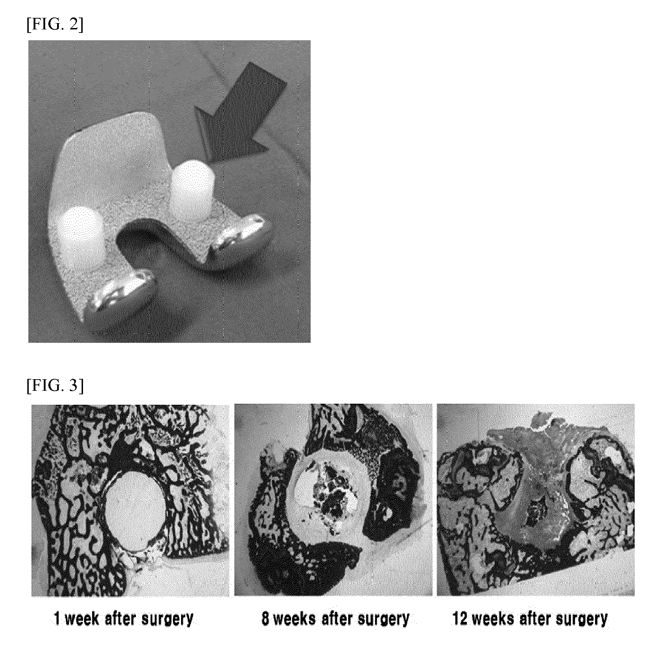

FIG. 2 is a photograph showing an artificial joint mounted with the implant manufactured in Example 1.

Referring to FIG. 2, the implant (arrow) mounted in the artificial joint (brand name: Optigen, manufactured by Yon & I Corp.) is accurately depicted.

<Example 2>

The biodegradable magnesium alloy of Preparation Example 1 was extruded at an extrusion temperature of 400°C, and the reduction ratio (extrusion ratio) of the section area of the biodegradable magnesium alloy before and after extrusion was fixed at 15:1. The extruded magnesium alloy was processed to such a degree that it can be transplanted into a rat, so as to manufacture an implant.

Test Example 1: Histological evaluation of bone formation using magnesium alloy implant

Male SD rats having a weight of 200 g were used as a test animal. An insertion hole was formed in the thighbone of the rat using an implant motor by means of a general method. The implant manufactured in Example 2 was inserted into the insertion hole. Thereafter, rats were sacrificed after 1 week, 8 weeks and 12 weeks, respectively, and a bone sample was collected using an electric saw for the purpose of histological analysis. The collected bone sample was fixed in 10% neutral formalin, decalcified by sodium citrate, dewatered and then fixed in paraffin to fabricate a test sample having a size of 4 - 6 *µ*m. The test sample was dyed with hematoxylin-eosin, and then the histological change of the dyed test sample was observed using an optical microscope. The results thereof are shown in FIG. 3.

As shown in FIG. 3, after 1 week, it can be observed that the boundary of the magnesium alloy implant rod manufactured in Example 2 was clearly distinguished from peripheral tissue. However, after 8 weeks, it was observed that bone was formed, and, after 12 weeks, it was observed that the magnesium alloy implant was filled with new bone and fibrous tissue.

Test Example 2: Molecular biological evaluation of bone formation using magnesium alloy implant

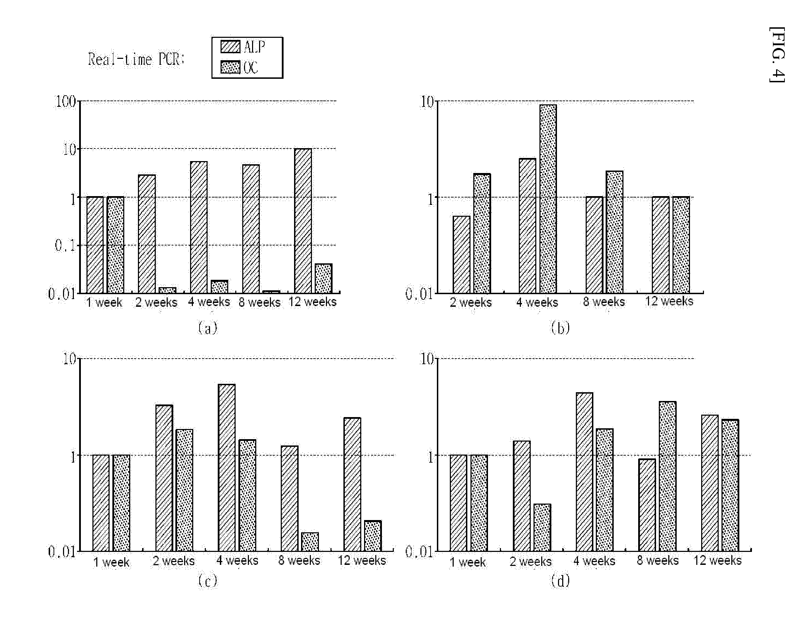

Male SD rats having a weight of 200 g were used as a test animal. Bone samples were collected in the same manner as in Test Example 1, except that a) autogeneous bone (pelvic bone of the rat), b) the magnesium alloy implant manufactured in Example 2, c) pure magnesium (manufactured by Timminco Metals Corp., raw material: MP21-31-31, composition: 99.98% ingot), and d) AZ91 (brand name) were inserted into the insertion hole, and that the rats were sacrificed after 1 week, 2 weeks, 4 weeks, 8 weeks and 12 weeks, respectively. mRNA was extracted from the bone sample using a general method, and the appearances of alkaline phosphatase (ALP) and osteocalcin (OC) indicating a bone forming index and a bone growth index were analyzed. The results thereof are shown in FIG. 4.

As shown in FIG. 4, it can be seen that the magnesium alloy implant exhibits a high rate of bone formation because the ALP and OC values of the magnesium alloy implant are higher than those of a) autogeneous bone, c) pure magnesium or d) AZ91.

From the test results, it can be clearly suggested that the magnesium alloy implant of the present invention can be used to improve the attachment of the implant to bone because the magnesium alloy implant is decomposed to induce the formation of new bone, and the decomposed portion of the magnesium alloy implant is replaced with new bone, so that the magnesium alloy implant is bonded with the bone, with the result that the strong bonding force between the implant and the bone can be maintained for a long period.

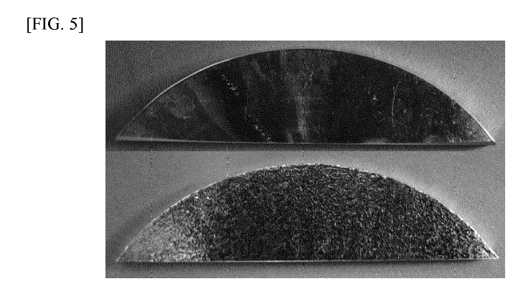

FIG. 5 is a photograph showing the surface of the biodegradable magnesium alloy of Preparation Example 2. Here, the biodegradable magnesium alloy located at the upper portion of FIG. 5 was not surface-treated, and the biodegradable magnesium alloy located at the lower portion of FIG. 5 was surface-treated.

Referring to FIG. 5, the biodegradable magnesium alloy of Preparation Example 2, which was not surface-treated, is glossy, and the biodegradable magnesium alloy of Preparation Example 2, which was surface-treated, is not glossy. Here, the surface treatment of the biodegradable magnesium alloy was conducted by shot peening.

As described above, the implant of the present invention fixes an artificial joint in bone and improves the initial bonding force and interfacial force between the artificial joint and the bone. Further, the implant of the present invention is bonded with bone and then decomposed, thus inducing the creation of bone. Since the implant of the present invention induces the creation of bone, strong bonding force between an artificial joint and bone can be maintained for a long period.

## Claims

1. An implant, which is used to fix an artificial joint in bone, comprising a biodegradable magnesium alloy.

2. The implant according to claim 1, wherein the implant is formed in the shape of any one selected from a screw, a bolt, a pin, a nail and a washer.

3. The implant according to claim 1, wherein the artificial joint is used as an artificial hip joint, an artificial knee joint, an artificial shoulder joint, an artificial elbow joint, an artificial wrist joint, an artificial ankle joint, an artificial finger joint or an artificial toe joint.

4. The implant according to claim 1, wherein the implant comprises a porous structure.

5. The implant according to claim 4, wherein the biodegradable magnesium alloy is located in a pore of the porous structure.

6. The implant according to claim 1, wherein the biodegradable magnesium alloy is represented by the following Formula 1:
Mg-A (1)
wherein A is at least one selected from the group consisting of manganese (Mn), cobalt (Co), nickel (Ni), chromium (Cr), copper (Cu), cadmium (Cd), zirconium (Zr), silver (Ag), gold (Au), palladium (Pd), platinum (Pt), rhenium (Re), iron (Fe), zinc (Zn), molybdenum (Mo), niobium (Nb), tantalum (Ta), titanium (Ti), strontium (Sr), silicon (Si), phosphorus (P) and selenium (Se).

7. The implant according to claim 1, wherein the biodegradable magnesium alloy is represented by the following Formula 2:
MgₐCa_{b}X_{c} (2)
wherein a, b and c are each independently a molar ratio of each component, a+b+c=1, 0.5≤a<1, 0≤b≤0.4, 0≤c≤0.4, and when at least one of b and c is more than 0 and c is 0, calcium (Ca) is included in an amount of 5 ~ 33 wt% based on a total amount of the biodegradable magnesium alloy; and
X is at least one selected from the group consisting of zirconium (Zr), molybdenum (Mo), niobium (Nb), tantalum (Ta), titanium (Ti), strontium (Sr), chromium (Cr), manganese (Mn), zinc (Zn), silicon (Si), phosphorus (P), nickel (Ni) and iron (Fe).

8. The implant according to claim 1, wherein the biodegradable magnesium alloy includes magnesium (Mg) and an impurity, the impurity is any one selected from the group consisting of manganese (Mn), iron (Fe), nickel (Ni) and mixtures thereof, an amount of the impurity is 1 part by weight or less but exceeding 0 parts by weight based on 100 parts by weight of magnesium (Mg), and a ratio of any one selected from the group consisting of iron (Fe), nickel (Ni) and mixtures thereofto manganese (Mn) is 5 or less but exceeding 0.

9. The implant according to claim 1, wherein the biodegradable magnesium alloy is represented by the following Formula 3, and comprises 23 wt% or less but exceeding 0 wt% of Ca; 10 wt% or less but exceeding 0 wt% ofY; and a balance of Mg:
Mg-Ca-Y (3)
wherein Y is Mn or Zn.

10. The implant according to claim 1, wherein the implant further comprises a bone forming agent.

11. The implant according to claim 1, wherein a surface of the biodegradable magnesium alloy is coated with at least one of a ceramic and a polymer.
